# EUROPEAN PATENT APPLICATION

(11) **EP 3 750 568 A1**
(43) Date of publication of application: **16.12.2020**
(21) Application number: 20182856.3
(22) Date of filing: 05.09.2013
(51) Int. Cl.: A61L 27/26, C08B 37/08, C08H 1/00, C08L 5/08, C08L 89/00

(54) **HYALURONIC ACID/COLLAGEN-BASED DERMAL FILLER COMPOSITIONS AND METHODS FOR MAKING SAME**

(30) Priority: 06.09.2012 US 201213605656
(62) Divisional of application: 13762683.4
(71) Applicant: ALLERGAN, INC., Irvine, CA 92612 (US)
(72) Inventor: Pollock, Jacob F., Charleston, WV West Virginia 25314 (US); Yu, Xiaojie, Irvine, CA California 92602 (US); Manesis, Nicholas J., Summerland, CA California 93067 (US)
(74) Representative: Hoffmann Eitle

(57) **Abstract**

The present invention provides a non-therapeutic method of enhancing or regenerating an anatomic feature of a human being, the method comprising: injecting or implanting an enhancement or regeneration device into a tissue of the human being to thereby enhance or regenerate at least a portion of the anatomic feature, wherein the enhancing or regenerating device comprises a crosslinked macromolecular matrix having a hyaluronic acid component and a collagen component.

## Description

### RELATED APPLICATION

This application claims priority to U.S. Provisional Patent Application No. 61/531,533, filed on September 6, 2011, and is also a continuation-in-part of U.S. Patent Application No. 13/603,213, which claims priority to U.S. Provisional Patent Application No. 61/531,533, filed September 6, 2011, the entire disclosures of which are incorporated herein by this specific reference.

### BACKGROUND

The present invention generally relates to dermal filler compositions, and more specifically relates to injectable dermal filler compositions including crosslinked hyaluronic acid and collagen. Hyaluronic acid and collagen are key structural components of human tissues. These biopolymers have been widely used to construct tissue engineering scaffolds and materials for cell culturing and regenerative medicine.

Skin aging is a progressive phenomenon, occurs over time and can be affected by lifestyle factors, such as alcohol consumption, tobacco and sun exposure. Aging of the facial skin can be characterized by atrophy, slackening, and fattening. Atrophy corresponds to a massive reduction of the thickness of skin tissue. Slackening of the subcutaneous tissues leads to an excess of skin and ptosis and leads to the appearance of drooping cheeks and eye lids. Fattening refers to an increase in excess weight by swelling of the bottom of the face and neck. These changes are typically associated with dryness, loss of elasticity, and rough texture.

Hyaluronan, also known as hyaluronic acid (HA) is a non-sulfated glycosaminoglycan that is distributed widely throughout the human body in connective, epithelial, and neural tissues. Hyaluronan is abundant in the different layers of the skin, where it has multiple functions such as, e.g., to ensure good hydration, to assist in the organization of the extracellular matrix, to act as a filler material; and to participate in tissue repair mechanisms. However, with age, the quantity of hyaluronan, collagen, elastin, and other matrix polymers present in the skin decreases. For example, repeated exposed to ultra violet light, e.g., from the sun, causes dermal cells to both decrease their production of hyaluronan as well as increase the rate of its degradation. This hyaluronan loss results in various skin conditions such as, e.g., imperfects, defects, diseases and/or disorders, and the like. For instance, there is a strong correlation between the water content in the skin and levels of hyaluronan in the dermal tissue. As skin ages, the amount and quality of hyaluronan in the skin is reduced. These changes lead to drying and wrinkling of the skin.

Dermal fillers are useful in treating soft tissue condition and in other skin therapies because the fillers can replace lost endogenous matrix polymers, or enhance/facilitate the function of existing matrix polymers, in order to treat these skin conditions. In the past, such compositions have been used in cosmetic applications to fill wrinkles, lines, folds, scars, and to enhance dermal tissue, such as, e.g., to plump thin lips, or fill-in sunken eyes or shallow cheeks. Earlier dermal filler products generally were made of collagens. One common matrix polymer used in modern dermal filler compositions is hyaluronan. Because hyaluronan is natural to the human body, it is a generally well tolerated and a fairly low risk treatment for a wide variety of skin conditions.

Originally, compositions comprising hyaluronan where made from naturally-occurring polymers, which exist in an uncrosslinked state. Although exhibiting excellent biocompatibility and affinity for water molecules, naturally-occurring hyaluronan exhibits poor biomechanical properties as a dermal filler. One primary reason is that because this polymer is uncrosslinked, it is highly soluble and, as such, is cleared rapidly when administered into a skin region. This *in vivo* clearance is primarily achieved by rapid degradation of the polymers, principally enzymatic degradation via hyaluronidase and chemical degradation via free-radicals. Thus, while still in commercial use, compositions comprising uncrosslinked hyaluronan polymers tend to degrade within a few days after administration and thus require fairly frequent reinjection to maintain their skin improving effect.

To minimize the effect of these *in vivo* degradation pathways, matrix polymers are crosslinked to one another to form a stabilized hydrogel. Because hydrogels comprising crosslinked matrix polymers are a more solid substance, dermal fillers comprising such hydrogels remain in place at the implant site longer. In addition, these hydrogels are more suitable as a dermal filler because the more solid nature thereof improves the mechanical properties of the filler, allowing the filler to better lift and fill a skin region. Hyaluronan polymers are typically crosslinked with a crosslinking agent to form covalent bonds between hyaluronan polymers. Such crosslinked polymers form a less water soluble hydrogel network that is more resistant to degradation, and thus requires less frequent reinjection, than the non-crosslinked hyaluronan compositions.

The present invention provides new injectable dermal filler compositions for enhancing the appearance of skin.

### SUMMARY

Accordingly, new dermal filler compositions, as well as methods of making same, are provided. Some embodiments include homogeneous hydrogel compositions prepared from hyaluronic acid and collagen. These compositions may be prepared by a method comprising crosslinking hyaluronic acid and collagen. In some embodiments, the hyaluronic acid and collagen are crosslinked under conditions in which both components are initially soluble in aqueous solution.

Some embodiments include method of crosslinking hyaluronic acid and collagen comprising: dissolving a hyaluronic acid and a collagen in an aqueous solution to form an aqueous pre-reaction solution, wherein the aqueous pre-reaction solution further comprises a salt or has a low pH; and modifying the aqueous pre-reaction solution to form a crosslinking reaction mixture comprising: the hyaluronic acid; the collagen; a water soluble coupling agent; and the salt; and wherein the crosslinking reaction has a higher pH than the aqueous pre-reaction solution; and allowing the crosslinking reaction mixture to react to thereby crosslink the hyaluronic acid and the collagen.

Some embodiments include a method of crosslinking hyaluronic acid and collagen comprising: dissolving a hyaluronic acid and a collagen in an aqueous solution to form an aqueous pre-reaction solution, wherein the aqueous pre-reaction solution further comprises a salt or has a pH less than about 4; and modifying the aqueous pre-reaction solution to form a crosslinking reaction mixture comprising: the hyaluronic acid; the collagen; a water soluble carbodiimide; an N-hydroxysuccinimide or an N-hydroxysulfosuccinimide; and the salt; and wherein the crosslinking reaction has a higher pH than the aqueous pre-reaction solution; and allowing the crosslinking reaction mixture to react to thereby crosslink the hyaluronic acid and the collagen.

Some embodiments include a method of crosslinking hyaluronic acid and collagen comprising: dissolving a hyaluronic acid and a collagen in an aqueous solution to form an aqueous pre-reaction solution, wherein the aqueous pre-reaction solution further comprises a salt or has a pH less than about 4; and modifying the aqueous pre-reaction solution to form a crosslinking reaction mixture comprising: the hyaluronic acid; the collagen; a water soluble carbodiimide; an activating agent such as an N-hydroxysuccinimide; and the salt; and wherein the crosslinking reaction has a higher pH than the aqueous pre-reaction solution; and allowing the crosslinking reaction mixture to react to thereby crosslink the hyaluronic acid and the collagen.

Some embodiments include composition comprising: a hyaluronic acid; a collagen; and a water-soluble coupling agent; wherein the composition is an aqueous solution.

Some embodiments include composition comprising: a hyaluronic acid; a collagen; a water-soluble coupling agent; and a buffer; wherein the composition is an aqueous solution.

Some embodiments include a crosslinked macromolecular matrix comprising: a hyaluronic acid component and a collagen component; wherein the hyaluronic acid component is crosslinked to the collagen component by a crosslinking component; and wherein the crosslinking component comprises a plurality of crosslink units, wherein at least a portion of the crosslink units comprise an ester bond or an amide bond.

Some embodiments include a crosslinked macromolecular matrix comprising: a hyaluronic acid component; a collagen component derived from collagen type I or collagen type III; wherein the hyaluronic acid component is crosslinked to the collagen component by a crosslinking component; and wherein the crosslinking component comprises a plurality of crosslink units, wherein at least a portion of the crosslink units comprise an ester bond or an amide bond.

Some embodiments include a soft tissue aesthetic product composition comprising: an aesthetic device having a form suitable for injecting or implanting into human tissue; and a label comprising instructions to inject or implant the aesthetic device into human tissue; wherein the aesthetic device comprises a crosslinked macromolecular matrix described herein.

Some embodiments include a soft tissue enhancement or regeneration product composition comprising: an enhancement or regeneration device having a form suitable for injecting or implanting into human tissue; and a label comprising instructions to inject or implant the enhancement or regeneration device into human tissue; wherein the enhancement or regeneration device comprises a crosslinked macromolecular matrix described herein.

Some embodiments include a method of improving an aesthetic quality of an anatomic feature of a human being comprising: injecting or implanting an aesthetic device into a tissue of the human being to thereby improve the aesthetic quality of the anatomic feature; wherein the aesthetic device comprises a crosslinked macromolecular matrix composition described herein.

Some embodiments include a method of enhancing or regenerating an anatomic feature of a human being comprising: injecting or implanting an enhancement or regeneration device into a tissue of the human being to thereby enhance or regenerate at least a portion of the anatomic feature; wherein the enhancing or regenerating device comprises a crosslinked macromolecular matrix comprising described herein.

### BRIEF DESCRIPTION OF THE DRAWINGS

FIG.1A is a plot of a frequency sweep for the gel of Example 3.
FIG.1B is a plot of a strain sweep for the gel of Example 3.
FIG. 2 is an extrusion profile for the gel of Example 3.
FIG. 3 depicts scanning electron microscope images (SEM images) of the gel from Example 4 shown at 50X (A), 1,000X (B), and 40,000X (C).

### DETAILED DESCRIPTION

Crosslinked hyaluronic acid, collagen, and crosslinked collagen hydrogels, such as those used in dermal fillers, do not actively promote cellular infiltration and tissue in-growth. Similarly, collagen simply blended into hyaluronic acid hydrogels does not promote tissue integration or *de novo* tissue generation. However, some compositions described herein do promote cellular migration into the hydrogels and tissue formation within the gels when implanted *in vivo.*

Hyaluronic acid-collagen hydrogels may be synthesized by coupling a hyaluronic acid with a collagen using a coupling agent, such as a carbodiimide. In these hydrogels, hyaluronic acid may serve as a biocompatible water-binding component, providing bulk and isovolumetric degradation. Additionally, collagen may impart cell adhesion and signaling domains to promote cell attachment, migration, and other cell functions such as extra-cellular matrix deposition. The biopolymers form homogeneous hydrogels with tunable composition, swelling, and mechanical properties. Compositions can be made to be injectable for minimally invasive implantation through syringe and needle.

Hyaluronic acid is a non-sulfated glycosaminoglycan that enhances water retention and resists hydrostatic stresses. It is non-immunogenic and can be chemically modified in numerous fashions. Hyaluronic acid may be anionic at pH ranges around or above the pKa of its carboxylic acid groups.

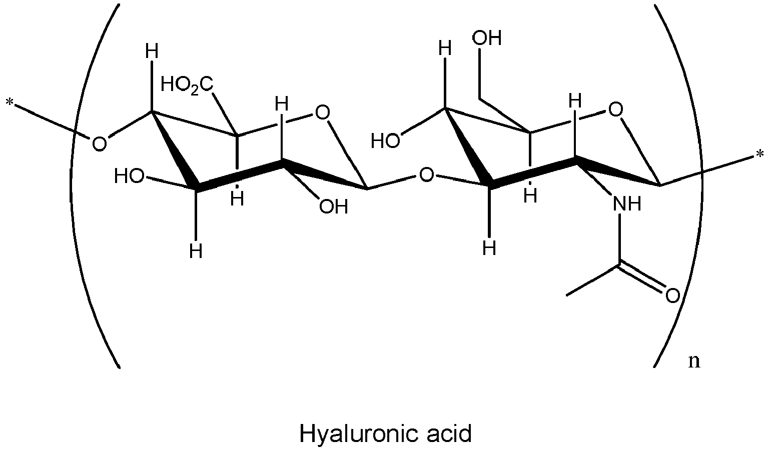

Collagen is a protein that forms fibrils and sheets that bear tensile loads. Collagen also has specific integrin-binding sites for cell adhesion and is known to promote cell attachment, migration, and proliferation. Collagen may be positively charged because of its high content of basic amino acid residues such as arginine, lysine, and hydroxylysine.

Because hyaluronic acid may be anionic and collagen may be cationic, the two macromolecules may form polyionic complexes in aqueous solution. A polyionic complex may be significantly less soluble in water than either hyaluronic acid or collagen, and thus may precipitate out of aqueous solution when the two macromolecules are together in a mixture. Furthermore, collagens are often soluble only at low pH and may precipitate from solution when brought to a pH amenable to carbodiimide coupling.

Under certain conditions, a hyaluronic acid and a collagen may be combined in an aqueous liquid in which both components are soluble. A hyaluronic acid and a collagen may then be crosslinked while both are dissolved in an aqueous solution to form a hydrogel. Reaction conditions such as the concentration of hyaluronic acid, the concentration of collagen, the pH of the solution, and salt concentration may be adjusted to help to prevent polyionic complex formation between anionic hyaluronic acid and cationic collagen. They may also help to prevent collagen microfibril formation, which results in precipitation from solution and may prevent crosslinking.

Some embodiments include a method of crosslinking hyaluronic acid and collagen. This method generally comprises a dissolution step which results in an aqueous pre-reaction solution. In a dissolution step, hyaluronic acid and collagen are dissolved in an aqueous solution that has a low pH and/or a salt to form an aqueous pre-reaction solution.

A hyaluronic acid-collagen crosslinking method further comprises an activation step. In an activation step, an aqueous pre-reaction solution is modified at least by adding a water soluble coupling agent and/or by increasing the pH of the solution. If needed, a salt may also be added to keep the hyaluronic acid and collagen in solution at the higher pH. Thus, a crosslinking reaction mixture comprises hyaluronic acid and collagen dissolved or dispersed in an aqueous medium, a water soluble coupling agent, and a salt, and has a higher pH than the aqueous pre-reaction solution from which it was derived. The crosslinking reaction mixture is allowed to react to thereby crosslink the hyaluronic acid and the collagen.

In some embodiments, the pH of the aqueous pre-reaction solution may be increased and a substantial amount of fiber formation may be allowed to occur in the solution before adding the water soluble coupling agent. In some embodiments, the water soluble coupling agent may be added to the aqueous pre-reaction solution before substantially any fiber formation occurs.

A crosslinking reaction mixture can react to form a crosslinked macromolecular matrix. Since reaction occurs in an aqueous solution, a crosslinked macromolecular matrix may be dispersed in an aqueous liquid in hydrogel form as it is formed by a crosslinking reaction. A crosslinked macromolecular matrix may be kept in hydrogel form because, in many instances, a crosslinked macromolecular matrix may be used in hydrogel form.

In some embodiments, an aqueous pre-reaction solution or a crosslinking reaction mixture may further comprise about 10% to about 90% of an organic solvent in which hyaluronic acid has poor solubility, such as ethanol, methanol, isopropanol, or the like.

After a crosslinking reaction has occurred, the crosslinked macromolecular matrix may be particulated or homogenized through a mesh. This may help to form an injectable slurry or hydrogel. A mesh used for particulating a crosslinked macromolecular matrix may have any suitable pore size depending upon the size of particles desired. In some embodiments, the mesh may have a pore size of about 10 microns to about 100 microns, about 50 microns to about 70 microns, or about 60 microns.

A hydrogel comprising a crosslinked molecular matrix may be treated by dialysis for sterilization or other purposes. Dialysis may be carried out by placing a semipermeable membrane between the hydrogel and another liquid so as to allow the hydrogel and the liquid to exchange molecules or salts that can pass between the membrane.

A dialysis membrane may have a molecular weight cutoff that may vary. For example, the cutoff may be about 5,000 daltons to about 100,0000 daltons, about 10,000 daltons to about 30,000 daltons, or about 20,000 daltons.

The dialysis may be carried out against a buffer solution, meaning that the liquid on the other side of the membrane from the hydrogel may be a buffer solution. In some embodiments, the buffer solution may be a sterile phosphate buffer solution that may comprise phosphate buffer, potassium chloride, and/or sodium chloride. A sterile phosphate buffer solution may be substantially isosmotic with respect to human physiological fluid. Thus, when dialysis is complete, the liquid component of a hydrogel may be substantially isosmotic with respect to human physiological fluid.

In some embodiments, a crosslinked macromolecular complex may further comprise an aqueous liquid. For example, the crosslinked macromolecular complex may absorb the aqueous liquid so that a hydrogel is formed. An aqueous liquid may comprise water with a salt dissolved in it, such as a phosphate buffer, sodium chloride, potassium chloride, etc. In some embodiments, an aqueous liquid may comprise water, sodium chloride at a concentration of about 100 mM to about 200 mM, potassium chloride at a concentration of about 2 mM to about 3 mM, and phosphate buffer at a concentration of about 5 mM to about 15 mM, wherein the pH of the liquid is about 7 to about 8.

A hydrogel may be used in a soft tissue aesthetic product. An aesthetic product includes any product that improves any aesthetic property of any part of an animal or human being. A soft tissue aesthetic product may comprise: an aesthetic device having a form suitable for injecting or implanting into human tissue; and a label comprising instructions to inject or implant the aesthetic component into human tissue; wherein the aesthetic device comprises a crosslinked macromolecular matrix described herein. Some products may comprise the crosslinked macromolecular matrix in hydrogel form.

Some embodiments include a method of improving an aesthetic quality of an anatomic feature of a human being. Improving an aesthetic quality of an anatomic feature of a human being includes improving any kind of aesthetic quality including appearance, tactile sensation, etc., and improving any anatomical feature, including those of the face, limbs, breasts, buttocks, etc. Such a method may comprise: injecting or implanting an aesthetic device into a tissue of the human being to thereby improve the aesthetic quality of the anatomic feature; wherein the aesthetic device comprises a crosslinked macromolecular matrix composition described herein. In some embodiments, the crosslinked macromolecular matrix used in the product may be in hydrogel form.

In some embodiments, a hydrogel of a crosslinked macromolecular complex may have a storage modulus of about 1 Pa to about 10,000 Pa, about 50 Pa to 10,000 Pa, about 500 Pa to about 1000 Pa, about 500 Pa to about 5000 Pa, about 850 Pa, about 852 Pa, about 560 Pa, about 556 Pa, about 1000 Pa, or any value in a range bounded by, or between, any of these values.

In some embodiments, a hydrogel of a crosslinked macromolecular complex may have a loss modulus of about 1 Pa to about 500 Pa, about 10 Pa to 200 Pa, about 100 Pa to about 200 Pa, about 20 Pa, about 131 Pa, about 152 Pa, or any value in a range bounded by, or between, any of these values.

In some embodiments, a hydrogel of a crosslinked macromolecular complex may have an average extrusion force of about 10 N to about 50 N, about 20 N to 30 N, or about 25 N, when the hydrogel is forced through a 30G needle syringe by moving the plunger of a 1 mL syringe containing the hydrogel at a rate of 100 mm/min for about 11 mm, and measuring the average force from about 4 mm to about 10 mm.

A crosslinked macromolecular matrix may have tunable swelling properties based on reaction conditions and hydrogel dilution. In some embodiments, a crosslinked macromolecular matrix may have a swelling ratio of about 20 to about 200. A swelling ratio is the ratio of the weight of the crosslinked macromolecular matrix after synthesis to the weight of the crosslinked macromolecular matrix without any water. The crosslinked macromolecular matrix may have a swelling power of about 1 to about 7. The swelling power is the ratio of the weight of the crosslinked macromolecular matrix when it is saturated with water to the weight of the crosslinked macromolecular matrix after synthesis.

In a crosslinking reaction, the molecular weight of a hyaluronic acid may vary. In some embodiments, a hyaluronic acid may have a molecular weight of about 200,000 daltons to about 10,000,000 daltons, about 500,000 daltons to about 10,000,000 daltons, about 1,000,000 daltons to about 5,000,000 daltons, or about 1,000,000 daltons to about 3,000,000 daltons. When the crosslinking reaction occurs, the resulting crosslinked macromolecular product may have a hyaluronic acid component derived from the hyaluronic acid in the crosslinking reaction. Thus, the ranges recited above may also apply to the molecular weight of a hyaluronic acid component, e.g. about 200,000 daltons to about 10,000,000 daltons, about 500,000 daltons to about 10,000,000 daltons, about 1,000,000 daltons to about 5,000,000 daltons, or about 1,000,000 daltons to about 3,000,000 daltons. The term "molecular weight" is applied in this situation to a portion of the matrix even though the hyaluronic acid component may not actually be a separate molecule due to the crosslinking. In some embodiments, a higher molecular weight hyaluronic acid may result in a crosslinked molecular matrix that may have a higher bulk modulus and/or less swelling.

The concentration of hyaluronic acid in an aqueous pre-reaction solution or a crosslinking reaction mixture may vary. In some embodiments, hyaluronic acid is present at about 3 mg/mL to about 100 mg/mL, about 6 mg/mL to about 24 mg/mL, about 1 mg/mL to about 30 mg/mL, about 6 mg/mL, about 12 mg/mL, about 16 mg/mL, or about 24 mg/mL In some embodiments, higher hyaluronic acid concentration may lead to higher stiffness and/or more swelling in the crosslinked macromolecular matrix.

Any type of collagen may be used in the methods and compositions described herein. In some embodiments, collagen type I, collagen type III, collagen type IV, collagen type VI, or a combination thereof, may be used. A collagen may be derived from cell culture, animal tissue, or recombinant means, and may be derived from human, porcine, or bovine sources. Some embodiments comprise collagen derived from human fibroblast culture. Some embodiments comprise collagen that has been denatured to gelatin.

Collagen concentration in an aqueous pre-reaction solution or a crosslinking reaction mixture may vary. In some embodiments, collagen may be present at a concentration of about 1 mg/mL to about 40 mg/mL, about 1 mg/mL to about 15 mg/mL, about 3 mg/mL to about 12 mg/mL, about 1.7 mg/mL, about 3 mg/mL, about 6 mg/mL, about 8 mg/mL, or about 12 mg/mL.

In some embodiments, the weight ratio of hyaluronic acid to collagen in a aqueous pre-reaction solution or a aqueous pre-reaction solution or a crosslinking reaction mixture (e.g. [wt hyaluronic acid]/[wt collagen]) may be about 0.5 to about 7, about 1 to about 5, or about 1 to about 3, or about 1 to about 2, or about 1, or about 2. When the crosslinking reaction occurs, the resulting crosslinked macromolecular product may have a collagen component derived from the collagen in the crosslinking reaction. Thus, the resulting crosslinked macromolecular matrix may have a weight ratio of hyaluronic acid component to collagen component that corresponds to the weight ratio in the crosslinking reaction, e.g. about 0.5 to about 7, about 1 to about 3, about 1 to about 2, about 1, or about 2. A higher weight ratio of hyaluronic acid to collagen may result in a crosslinked macromolecular matrix with increased swelling, decreased stiffness, and/or decreased cell adhesion.

In increase in the amount of both hyaluronic acid and collagen may result in a crosslinked macromolecular matrix with increased stiffness.

A salt may help to screen the negative charges of hyaluronic acid from positive charges of collagen, and may thus prevent precipitation of a polyionic ion complex from solution. However, high concentrations of salt may reduce the solubility of some components in solution. Thus, in some embodiments, the salt concentration of an aqueous pre-reaction solution or a crosslinking reaction mixture may be high enough to screen the charges so that the polyionic ion complex is not formed, but also low enough so that the components of the mixture remain in solution. For example, the total salt concentration of some aqueous pre-reaction solutions or crosslinking reaction mixtures may be about 10 mM to about 1 M, about 100 mM to about 300 mM, or about 150 mM. In some embodiments, a higher salt concentration may increase the efficiency of a crosslinking reaction, which may result in lower swelling and/or higher stiffness.

Some salts in an aqueous pre-reaction solution or a crosslinking reaction mixture may be non-coordinating buffers. Any non-coordinating buffer may be used that is capable of buffering the mixture and does not form coordinating complexes with coupling agents or metal atoms. Examples of suitable non-coordinating buffers may include, but are not limited to, 2-(N-morpholino)ethanesulfonic acid (MES), 3-(N-morpholino)propanesulfonic acid (MOPS), 4-(2-hydroxyethyl)-1-piperazinyl)ethanesulfonic acid (HEPES), 3-[4-(2-hydroxyethyl)-1-piperazinyl]propanesulfonic acid (HEPPS), N-cyclohexyl-2-aminoethanesulfonic acid (CHES), N-cyclohexyl-3-aminopropanesulfonic acid (CAPS), etc.

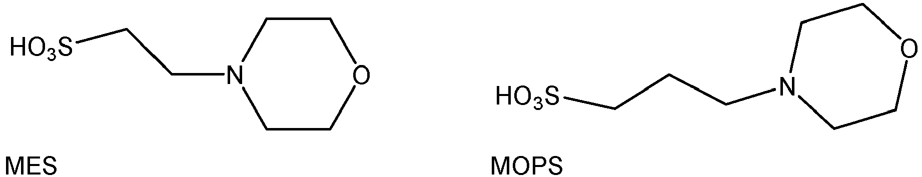

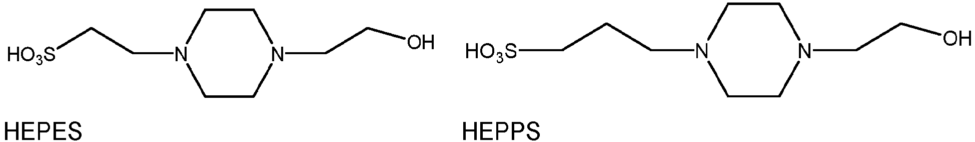

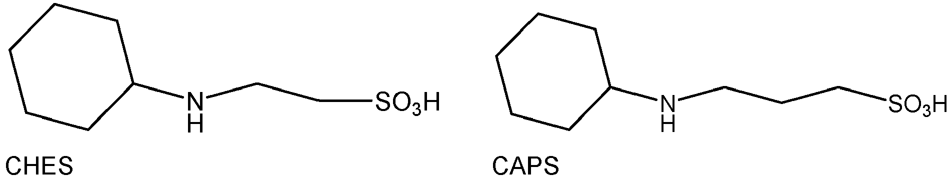

The concentration of a non-coordinating buffer may vary. For example, some aqueous pre-reaction solutions or crosslinking reaction mixtures may have a buffer concentration in a range of about 10 mM to about 1 M, about 10 mM to about 500 mM, about 20 mM to about 100 mM, or about 25 mM to about 250 mM. Some aqueous pre-reaction solutions or crosslinking reaction mixtures comprise MES at a concentration of about 20 mM to about 200 mM, about 20 mM to about 100 mM, about 100 mM, or about 180 mM.

Non-buffering salts may also be included in an aqueous pre-reaction solution or a crosslinking reaction mixture as an alternative to, or in addition, to buffering salts. Some examples may include inorganic salts such as sodium chloride, potassium chloride, lithium chloride, potassium bromide, sodium bromide, lithium bromide, and the like. The concentration of a non-buffering salt may vary. For example, some mixtures may have a non-buffering salt concentration in a range of about 10 mM to about 1 M, about 30 mM to about 500 mM, or about 50 mM to about 300 mM. In some embodiments, sodium chloride may be present at a concentration in a range of about 0.5 % w/v to about 2 % about 0.9 % w/v, about 1.6 % w/v, about 20 mM to about 1 M, about 40 mM to about 500 mM, about 50 to 300 mM, about 80 mM to about 330 mM, about 150 mM, or about 270 mM.

The pH of an aqueous pre-reaction solution may be lower than the pH of a crosslinking reaction mixture. If the salt content of the aqueous pre-reaction solution is low, the pH may be lower to enhance solubility of the hyaluronic acid and the collagen. If the salt content is higher, the pH may be higher in the aqueous pre-reaction solution. In some embodiments, the pH of the aqueous pre-reaction mixture is about 1 to about 8, about 3 to about 8, about 4 to about 6, about 4.7 to about 7.4, or about 5.4. For low salt concentrations, the pH may be about 1 to about 4 or about 1 to about 3. In some embodiments, a pH of around 5.4 may result in a crosslinked macromolecular matrix having higher stiffness and/or lower swelling.

In some embodiments, pH may be adjusted to neutral to allow collagen gelation or fiber formation before adding a coupling agent.

In some embodiments, the pH may be adjusted to neutral immediately prior to, around the time of, or after adding a coupling agent, such that collagen gelation is reduced or does not substantially occur.

Any water-soluble coupling agent may be used that can crosslink hyaluronic acid to collagen. Some non-limiting examples of a coupling agent include carbodiimides such as N,N'-dicyclohexylcarbodiimide (DCC), N,N'-diisopropylcarbodiimide (DIC), or 1-ethyl-3-(3-dimethylaminopropyl)carbodiimide (EDC), etc. Carbodiimide coupling agents may facilitate ester or amide bond formation without becoming part of the linkage. In other words, an ester bond or an amide bond may comprise atoms from a carboxylate group from one of hyaluronic acid or collagen, and a hydroxyl group or an amine group from the other. However, other coupling agents that become part of the crosslinking group may be used. The concentration of a coupling agent may vary. In some embodiments, a coupling agent may be present at about 2 mM to about 150 mM, about 2 mM to about 50 mM, about 20 mM to about 100 mM, or about 50 mM. In some embodiments, the coupling agent is EDC that is present at a concentration of about 20 mM to about 100 mM, about 2 mM to about 50 mM, or about 50 mM. Increasing the carbodiimide concentration up to about 50 mM may result in a crosslinked macromolecular matrix with greater hydrogel stiffness and/or less swelling.

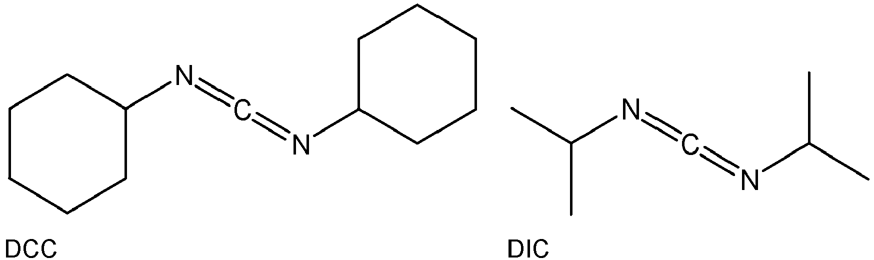

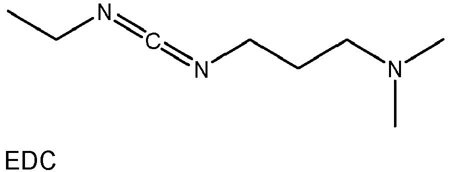

A crosslinking reaction includes any reaction hyaluronic acid is covalently linked to collagen in a plurality of (e.g. more than 1) positions. In some embodiments, a crosslinking reaction may be represented by Scheme 1 below.

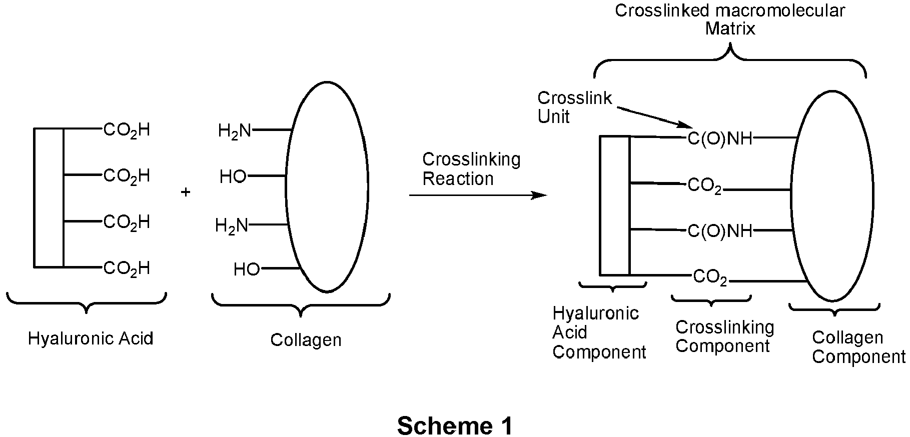

In Scheme 1, only some of the reacting functional groups are depicted, and many functional groups which may react in a crosslinking reaction, but may also remain unreacted, are not shown. For example, OH, CO₂H, -NHCOCH₃, and other groups on hyaluronic acid that are not shown may react, but may also remain unreacted. Similarly, collagen may have additional groups that may react, but may also remain unreacted, such as OH, SH, CO₂H, NH₂, etc. Additionally, fewer groups may react than those depicted.

In Scheme 1, functional groups such as CO₂H on hyaluronic acid may react with functional groups on collagen such as NH₂ and OH to form several crosslink units. The crosslink units together make up the crosslinking component. In Scheme 1, a coupling component does not become part of a crosslink unit. However, for some coupling agents, at least part of a coupling agent may incorporated into a crosslink unit. The hyaluronic acid component includes hyaluronic acid that has reacted to become part of a crosslinked macromolecular matrix. The collagen component includes collagen that has reacted to become part of a crosslinked macromolecular matrix. In addition to the crosslinking between hyaluronic acid and collagen, hyaluronic acid or collagen may be partially self-crosslinked. Thus, Scheme 1 is presented for convenience in understanding the crosslinking reaction, but does not necessarily reflect an actual chemical structure. For example, a crosslinked molecular matrix may be a network of hyaluronic acid macromolecules and collagen macromolecules, with many macromolecules crosslinked to more than one macromolecule.

As a result of a crosslinking reaction, a crosslinked macromolecular matrix may comprise a crosslinking component that crosslinks or covalently connects the hyaluronic acid component to the collagen component. As explained above, a crosslink component comprises a plurality of crosslink units, or individual covalent bonding links, between the hyaluronic acid component and the collagen component. A crosslink unit may simply be a direct bond between a hyaluronic acid component and a collagen components, so that the coupling agent may not be incorporated into the crosslinked macromolecular matrix. Alternatively, a crosslink unit may contain additional atoms or groups from the coupling agent such that at least a portion of the coupling agent may become part of the crosslinked macromolecular matrix. At least a portion of the crosslink units may comprise an ester bond or an amide bond. In some embodiments, at least a portion of the crosslink units may be -CON- or -CO₂-, where the N is a nitrogen from an amino acid residue.

An activating agent may be used to increase the rate of the crosslinking reaction and the number of crosslink units in the final product. In some embodiments, an activating agent may be a triazole such as hydroxybenzotriazole (HOBT) or 1-hydroxy-7-azabenzotriazole (HOAT); a fluorinated phenol such as pentafluorophenol; a succinimide such as N-hydroxysuccinimide (NHS) or N-hydroxysulfosuccinimide (sulfoNHS), and the like.

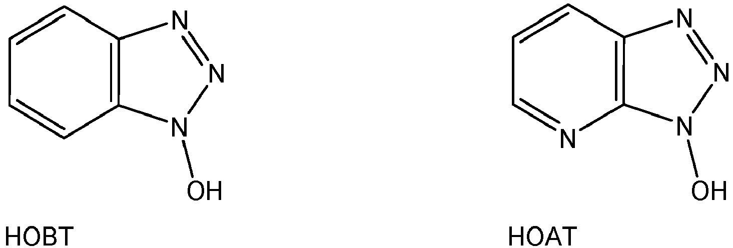

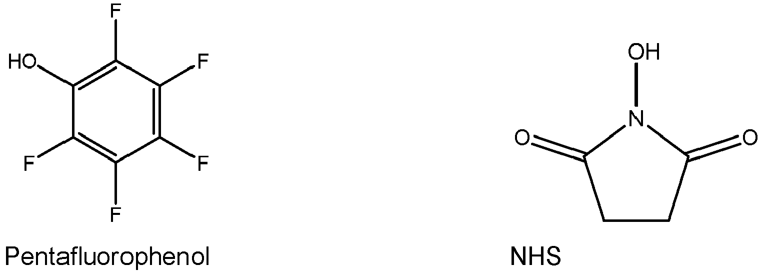

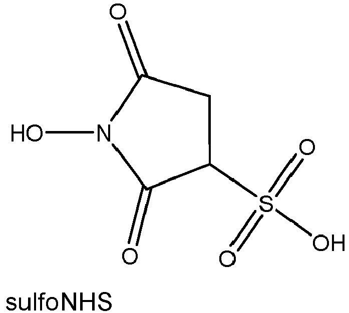

The concentration of an activating agent may vary. In some embodiments, the activating agent may have a concentration of about 2 mM to about 200 mM, about 2 mM to about 50 mM, about 20 mM to about 100 mM, or about 50 mM. In some embodments, the activating agent may be NHS or sulfoNHS is at a concentration of about 2 mM to about 50 mM. In some embodiments, the activating agent may be N-hydroxysulfosuccinimide, sodium salt, at a concentration of about 20 mM to about 100 mM, or about 50 Mm.

In some embodiments, a crosslinking reaction mixture may comprise a carbodiimide coupling agent and an activating agent. In some embodiments, the coupling agent is EDC and the activating agent is NHS or sulfoNHS. In some embodiments EDC is present at a concentration of about 2 mM to about 50 mM and NHS or sulfoNHS is present at about 2 mM to about 50 mM.

In some embodiments, a crosslinking reaction mixture may comprise hyaluronic acid at a concentration of about 3 mg/mL, human collagen type III at a concentration of about 3 mg/mL, 2-(N-morpholino)ethanesulfonic acid at a concentration of about 100 mM, sodium chloride at a concentration of about 0.9 wt% or about 150 mM, 1-ethyl-3-(3-dimethylaminopropyl)carbodiimide at a concentration of about 50 mM, and N-hydroxysulfosuccinimide sodium salt at a concentration of about 50 mM, wherein the solution has a pH of about 5.4.

In some embodiments, a crosslinking reaction mixture may comprise hyaluronic acid at a concentration of about 6 mg/mL, human collagen type III at a concentration of about 6 mg/mL, 2-(N-morpholino)ethanesulfonic acid at a concentration of about 180 mM, sodium chloride at a concentration of about 0.9 wt% or about 150 mM, 1-ethyl-3-(3-dimethylaminopropyl)carbodiimide at a concentration of about 50 mM, and N-hydroxysulfosuccinimide sodium salt at a concentration of about 50 mM, wherein the solution has a pH of about 5.4.

In some embodiments, a crosslinking reaction mixture may comprise hyaluronic acid at a concentration of about 16 mg/mL of, rat collagen type I at a concentration of about 8 mg/mL, 2-(N-morpholino)ethanesulfonic acid at a concentration of about 100 mM, sodium chloride at a concentration of about 0.9 wt% or about 150 mM, 1-ethyl-3-(3-dimethylaminopropyl)carbodiimide at a concentration of about 50 mM, and N-hydroxysulfosuccinimide sodium salt at a concentration of about 50 mM, wherein the solution has a pH of about 5.4.

In some embodiments, a crosslinking reaction mixture may comprise hyaluronic acid at a concentration of about 12 mg/mL, rat collagen type I at a concentration of about 12 mg/mL, 2-(N-morpholino)ethanesulfonic acid at a concentration of about 100 mM, sodium chloride at a concentration of about 0.9 wt% or about 150 mM, 1-ethyl-3-(3-dimethylaminopropyl)carbodiimide at a concentration of about 50 mM, and N-hydroxysulfosuccinimide sodium salt at a concentration of about 50 mM, wherein the solution has a pH of about 5.4.

In some embodiments, a crosslinking reaction mixture may comprise hyaluronic acid at a concentration of about 12 mg/mL, rat tail collagen type I at a concentration of about 12 mg/mL, 2-(N-morpholino)ethanesulfonic acid at a concentration of about 100 mM, sodium chloride at a concentration of about 0.9 wt% or about 150 mM, 1-ethyl-3-(3-dimethylaminopropyl)carbodiimide at a concentration of about 50 mM, and N-hydroxysulfosuccinimide sodium salt at a concentration of about 50 mM, wherein the solution has a pH of about 5.3.

In some embodiments, a crosslinking reaction mixture may comprise hyaluronic acid at a concentration of about 3 mg/mL, human collagen type I at a concentration of about 3 mg/mL, 2-(N-morpholino)ethanesulfonic acid at a concentration of about 100 mM, sodium chloride at a concentration of about 0.9 wt% or about 150 mM, 1-ethyl-3-(3-dimethylaminopropyl)carbodiimide at a concentration of about 50 mM, and N-hydroxysulfosuccinimide sodium salt at a concentration of about 50 mM, wherein the solution has a pH of about 5.4.

In some embodiments, a crosslinking reaction mixture may comprise hyaluronic acid at a concentration of about 12 mg/mL, human collagen type I at a concentration of about 6 mg/mL, 2-(N-morpholino)ethanesulfonic acid at a concentration of about 100 mM, sodium chloride at a concentration of about 0.9 wt% or about 150 mM, 1-ethyl-3-(3-dimethylaminopropyl)carbodiimide at a concentration of about 50 mM, and N-hydroxysulfosuccinimide sodium salt at a concentration of about 50 mM, wherein the solution has a pH of about 5.4.

In some embodiments, a crosslinking reaction mixture may comprise hyaluronic acid at a concentration of about 16 mg/mL, human collagen type I at a concentration of about 8 mg/mL, 2-(N-morpholino)ethanesulfonic acid at a concentration of about 100 mM, sodium chloride at a concentration of about 0.9 wt% or about 150 mM, 1-ethyl-3-(3-dimethylaminopropyl)carbodiimide at a concentration of about 50 mM, and N-hydroxysulfosuccinimide sodium salt at a concentration of about 50 mM, wherein the solution has a pH of about 5.4.

In some embodiments, a crosslinking reaction mixture may comprise hyaluronic acid at a concentration of about 12 mg/mL, human collagen type I at a concentration of about 12 mg/mL, 2-(N-morpholino)ethanesulfonic acid at a concentration of about 100 mM, sodium chloride at a concentration of about 0.9 wt% or about 150 mM, 1-ethyl-3-(3-dimethylaminopropyl)carbodiimide at a concentration of about 50 mM, and N-hydroxysulfosuccinimide sodium salt at a concentration of about 50 mM, wherein the solution has a pH of about 5.4.

In some embodiments, a crosslinking reaction mixture may comprise hyaluronic acid at a concentration of about 24 mg/mL, human collagen type I at a concentration of about 12 mg/mL, 2-(N-morpholino)ethanesulfonic acid at a concentration of about 100 mM, sodium chloride at a concentration of about 0.9 wt% or about 150 mM, 1-ethyl-3-(3-dimethylaminopropyl)carbodiimide at a concentration of about 50 mM, and N-hydroxysulfosuccinimide sodium salt at a concentration of about 50 mM, wherein the solution has a pH of about 5.4.

In some embodiments, a crosslinking reaction mixture may comprise hyaluronic acid at a concentration of about 16 mg/mL, collagen at a concentration of about 8 mg/mL, 2-(N-morpholino)ethanesulfonic acid at a concentration of about 100 mM, sodium chloride at a concentration of about 0.9 wt% or about 150 mM, 1-ethyl-3-(3-dimethylaminopropyl)carbodiimide at a concentration of about 50 mM, and N-hydroxysulfosuccinimide sodium salt at a concentration of about 50 mM, wherein the solution has a pH of about 5.4.

In some embodiments, a crosslinking reaction mixture may comprise hyaluronic acid at a concentration of about 1 mg/mL to about 20 mg/mL , porcine collagen type I at a concentration of about 1 mg/mL to about 15 mg/mL, 2-(N-morpholino)ethanesulfonic acid at a concentration of about 20 mM to about 200 mM, sodium chloride at a concentration of about 0.5 wt% to about 2 wt% or about 80 mM to about 330 mM, 1-ethyl-3-(3-dimethylaminopropyl)carbodiimide at a concentration of about 20 mM to about 100 mM, and N-hydroxysulfosuccinimide sodium salt at a concentration of about 20 mM to about 100 mM, wherein the solution has a pH of about 4 to about 6.

### Example 1

A solution of hyaluronic acid and collagen was created by dissolving 30 mg of 2 MDa hyaluronic acid sodium salt (Corneal) into 10 mL of 3 mg/mL collagen type III solution in 10 mM HCI (Fibrogen). 2-(N-morpholino) ethanesulfonic acid buffer salt (195.2 mg) was added to the solution along with 90 mg NaCl to form a pre-reaction solution at pH 2.5. The pH was then adjusted to 5.4 by addition of 200 µL 1 N NaOH. Next, 95.9 mg of 1-ethyl-3-(3-dimethylaminopropyl) carbodiimide HCI and 108.6 mg N-hydroxysulfosuccinimide sodium salt were added to the hyaluronic acid/collagen(III) solution and mixed thoroughly. The crosslinking reaction proceeded for 18 hrs before the gel was participated through a 60 micron pore-sized mesh. Following sizing, the gel was sterilized by dialysis through a 20 kDa molecular-weight cut-off cellulose ester membrane against 70 % isopropanol / 30 % water for 3 hrs at 4°C. Dialysis was then continued against sterile phosphate buffer for 72 hrs at 4°C with four changes of buffer. The gel was then dispensed into syringes under aseptic conditions.

### Example 2

A solution of hyaluronic acid was created by dissolving 60 mg of 2 MDa hyaluronic acid sodium salt (Corneal) in 20 mL of 100 mM 2-(N-morpholino) ethanesulfonic acid buffer with 0.9 wt% NaCl at pH 4.7. Upon full hydration and dissolution of the hyaluronic acid, this solution was mixed with 20 mL of 3 mg/mL human collagen(III) solution in 10 mM HCI (Fibrogen). The pH of the resulting hyaluronic acid/collagen(III) solution was adjusted to 5.4 with 1 N NaOH. The solution was then lyophilized to a dry sponge and reconstituted in 10 mL of distilled water to obtain a solution of 6 mg/mL hyaluronic acid and 6 mg/mL collagen(III). Next, 192 mg of 1-ethyl-3-(3-dimethylaminopropyl) carbodiimide HCI and 217 mg of N-hydroxysulfosuccinimide sodium salt were added to the hyaluronic acid/collagen(III) solution and mixed thoroughly. The crosslinking reaction proceeded for 18 hrs before the gel was particulated through a 60 micron pore-sized mesh. Following sizing, the gel was sterilized by dialysis through a 20 kDa molecular-weight cut-off cellulose ester membrane against 70 % isopropanol / 30 % water for 3 hrs at 4°C. Dialysis was then continued against sterile phosphate buffer for 72 hrs at 4°C with four changes of buffer. The gel was then dispensed into syringes under aseptic conditions.

### Example 3

Rat tail collagen(I) (Roche) was dissolved at 20 mg/mL in 0.01 N hydrochloric acid. Hyaluronic acid, 2 MDa molecular weight, (Corneal) was dissolved at 40 mg/mL in 100 mM 2-(N-morpholino) ethanesulfonic acid buffer salt (MES) buffer with 0.9 wt% NaCl at pH 4.7. MES buffer (500 mM) at pH 6.3 was made by dissolving 43 mg of NaCl and 95 mg MES buffer salt into 100 mM MES buffer with 0.9 wt% NaCl at pH 4.7. A pre-reaction solution was created by mixing 4.2 g of the rat collagen(I) solution, 4.2 g of the hyaluronic acid solution, and 1.05 mL of the MES buffer. An activating solution was made of 114 mg of N-hydroxysulfosuccinimide sodium salt in 530 µL of 100 mM MES buffer with 0.9 wt% NaCl at pH 5.2. A coupling solution was made of 100.6 mg of 1-ethyl-3-(3-dimethylaminopropyl) carbodiimide HCI in 530 µL of 100 mM MES buffer with 0.9 wt% NaCl at pH 5.2. The reaction mixture was then created by adding 500 µL of activating solution followed by 500 µL of coupling solution to 9 g of hyaluronic acid/collagen solution. The reaction mixture was transferred to a glass vial and centrifuged for 5 min at 4000 RPM to remove air bubbles. The reaction proceeded for 18 hrs at 4°C. The gel was then particulated through a 100 micron pore-sized mesh. Following sizing, the gel was sterilized by dialysis through a 20 kDa molecular-weight cut-off cellulose ester membrane against 70 % isopropanol / 30 % water for 3 hrs at 4°C. Dialysis was then continued against sterile phosphate buffer for 72 hrs at 4°C with four changes of buffer. The gel was then dispensed into syringes under aseptic conditions.

### Example 4

Rat tail collagen(I) in 0.01 N hydrochloric acid was concentrated from 5 mg/mL to 12 mg/mL using a centrifugal filtration device with 20 kDa molecular weight cutoff. Hyaluronic acid (120 mg, 2 MDa) was added to 10 mL of the collagen solution and allowed to hydrate for 60 minutes. The solution was then homogenized by passing from syringe to syringe through a leur-leur connector. Next, 90 mg of NaCl (0.9 wt%) and 200 mg of 2-(N-morpholino) ethanesulfonic acid buffer salt (100 mM) were added to the solution and mixed. Then 98 mg of 1-ethyl-3-(3-dimethylaminopropyl) carbodiimide HCI and 111 mg of N-hydroxysulfosuccinimide sodium salt (50 mM each) were added to the solution and quickly mixed. Finally, 200 µL of 1 N NaOH was added to the solution which was mixed by syringe-to-syringe passing. The reaction solution was transferred to a glass vial and centrifuged for 5 min at 4000 RPM to remove air bubbles. The reaction proceeded for 18 hrs at 4°C. The gel was then particulated through a 100 micron pore-sized mesh. Following sizing, the gel was sterilized by dialysis through a 20 kDa molecular-weight cut-off cellulose ester membrane against 70 % isopropanol / 30 % water for 3 hrs at 4°C. Dialysis was then continued against sterile phosphate buffer for 72 hrs at 4°C with four changes of buffer. The gel was then dispensed into syringes under aseptic conditions.

### Example 5

Oscillatory parallel plate rheology was used to characterize the mechanical properties of gels using an Anton Paar MCR 301. A plate diameter of 25 mm was used at a gap height of 1 mm. A frequency sweep from 0.1 to 10 Hz at a fixed strain of 2 % with logarithmic increase in frequency was applied followed by a strain sweep between 0.1 % and 300 % at a fixed frequency of 5 Hz with logarithmic increase in strain. The storage modulus (G') and loss modulus (G") were determined from frequency sweep measurements at 5 Hz.

The gel from Example 1 had a storage modulus (G') of 505 Pa and loss modulus (G") of 70 Pa.

The gel from Example 3 had a storage modulus (G') of 2,580 Pa and loss modulus (G") of 155 Pa.

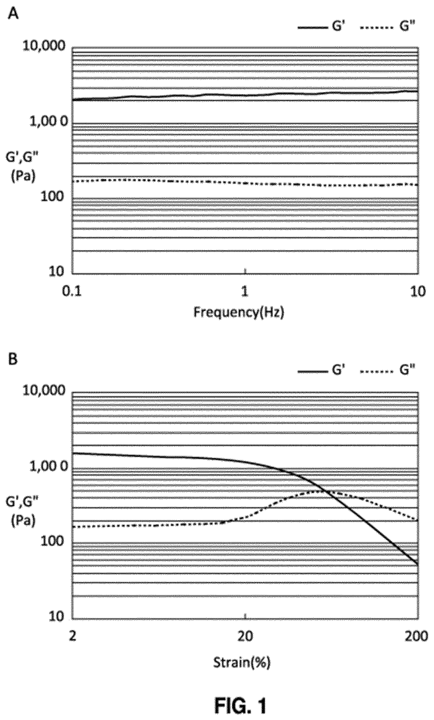

The frequency and strain sweeps for the gel from Example 3 are shown in Figure 1.

### Example 6

In order to determine the force required to extrude the gels, they were ejected from 1 mL BD syringes through 30G needles using an Instron 5564 with Bluehill 2 software. The plunger was pushed at a rate of 100 mm/min for 11.35 mm and the extrusion profile was recorded.

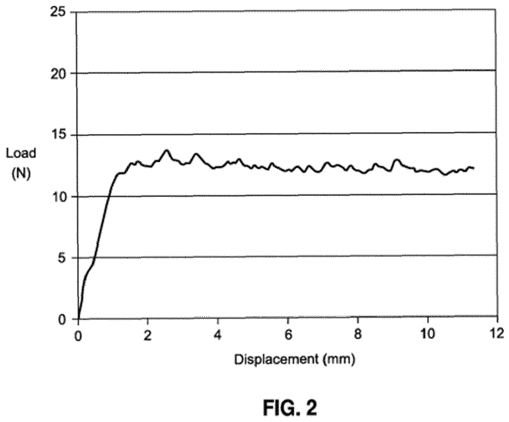

The extrusion profile through a 30G needle for gel from Example 3 is shown in Figure 2. The gel had an average extrusion force of 12.2 N from 4 through 10 mm.

### Example 7

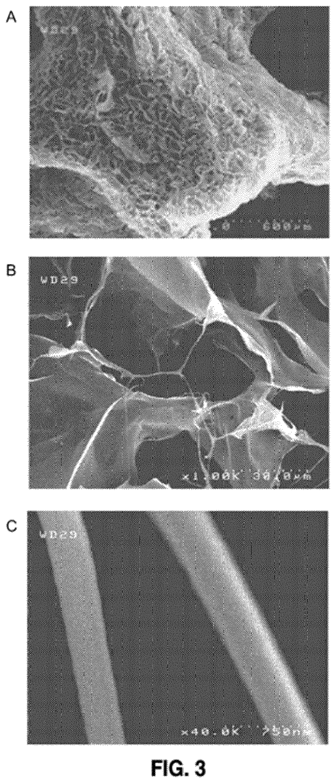

Gels were flash frozen in liquid nitrogen and dried by lyophilization. The dried sample was then imaged using a Hitachi S - 4500 scanning electron microscope (SEM). SEM images of the gel from Example 4 are shown in Figure 3 at 50X (A), 1,OOOX (B), and 40,000X (C). The fibrillar nature of collagen(I) is partially preserved in the hydrogel.

### Example 8

Rat tail collagen(I) in 0.01 N hydrochloric acid was concentrated from 5 mg/mL to 12 mg/mL using a centrifugal filtration device with 20 kDa molecular weight cutoff. Hyaluronic acid sodium salt (120 mg, 2 MDa) was added to 10 mL of the collagen solution and allowed to hydrate for 60 minutes. The solution was then homogenized by passing from syringe to syringe through a leur-leur connector, and 93 mg of NaCl, 201 mg of 2-(N-morpholino) ethanesulfonic acid buffer salt, and 200 µL of 1 N NaOH were added to the solution and mixed. 1-Ethyl-3-(3-dimethylaminopropyl) carbodiimide HCI (98 mg) and 111 mg of N-hydroxysulfosuccinimide sodium salt were then added and the final solution was mixed by syringe-to-syringe passing. The reaction solution was transferred to a glass vial and centrifuged for 5 min at 4000 RPM to remove air bubbles. The reaction proceeded for 16 hrs at 4°C. The gel was then particulated through a 60 micron pore-sized mesh. Following sizing, the gel was sterilized by dialysis through a 20 kDa molecular-weight cut-off cellulose ester membrane against 70 % isopropanol / 30 % water for 3 hrs at 4 °C. Dialysis was then continued against sterile phosphate buffer for 72 hrs at 4 °C with four changes of buffer. The gel was then dispensed into syringes under aseptic conditions.

### Example 9

The biocompatibility of gels was tested with a 50 µL intradermal sample injection in Sprague-Dawley rats. The implants were removed at 1 week and the explants were analyzed by histology with H&E and macrophage marker CD68 staining. Three 20X images of the CD68 staining were scored from 0-4 based on the degree of staining. These values were then averaged to give a sample score. Four samples for each gel were analyzed. The results of biocompatibility testing of Examples 3, 4, and 8 along with several commercially available dermal fillers are presented in Table 1.

**Table 1: CD68 staining scores for Examples 3, 4, and 8 as well as commercial dermal fillers.**

| | **average** | **stdev** |
|---|---|---|
| **Example 3** | 2.08 | 0.74 |
| **Example 4** | 2.58 | 0.63 |
| **Example 8** | 2.67 | 0.58 |
| **Juvederm® Ultra Plus** | 1.83 | 0.19 |
| **Juvederm® Voluma** | 1.92 | 0.42 |

### Example 10

Cytotoxicity of the gel from Example 4 was determined by NAMSA according to the Agarose Overlay Method of ISO 10993-5: Biological Evaluation of Medical Devices - Part 5: Tests for In Vitro Cytotoxicity. Triplicate wells were dosed with 0.1 mL of the gel placed on filter discs as well as 0.1 mL of 0.9 % NaCl solution placed on a filter discs and 1 cm length high density polyethylene as negative controls and 1 cm x 1 cm portion of latex as a positive control. Each was placed on an agarose surface directly overlaying a subconfluent monolayer of L929 mouse fibroblast cells. After incubating at 37 °C in 5 % CO₂ for 24 hours, the cultures were examined macroscopically and microscopically for any abnormal cell morphology and cell lysis. The test articles were scored from 0-4 based on the zone of cell lysis in proximity to the sample.

The gel from Example 4 showed no evidence of causing any cell lysis or toxicity and scored a 0 grade for cytotoxicity.

### Example 11

The intracutaneous reactivity of gel in rabbits from Example 4 was evaluated by NAMSA according to ISO 10993-10L Biological Evaluation of Medical Devices - Part 10: Tests for Irritation and Delayed-Type Hypersensitivity. Extract of the gel was prepared in 0.9 % NaCl solution (4:20 gel:saline ratio) and 0.2 mL of extract was injected intracutaneously into five separate sites on the right side of the back of each of three animals. The extract control was similarly injected on the left side of the back of each animal. The injection sites were observed at 24, 48, and 72 hours after injection for signs of erythema and edema. Erythema and edema were each scored on a scale of 0-4 at each site for each time point on each animal. The overall mean score was determined by dividing the sum of the scores by the total number of scores.

The gel from Example 4 had an overall mean score of 0, the same as the overall control group score. This indicated no signs of erythema or edema from the gel extract.

### Example 12

Hyaluronic acid sodium salt, 2 MDa molecular weight, was dissolved in human collagen(I) solution in 0.01 N hydrochloric acid (Advanced BioMatrix). Sodium chloride was added at 0.9 wt % and MES was added at 100 mM to the solution and mixed. The hyaluronic acid was allowed to hydrate for 1 hr and the solution was homogenized by syringe-to-syringe mixing. The pH of the solution was adjusted to 5.4 by addition of 1 N sodium hydroxide. 1-Ethyl-3-(3-dimethylaminopropyl) carbodiimide HCI (50 mM) and N-hydroxysulfosuccinimide sodium salt (50 mM) were added to the hyaluronic acid / collagen solution and quickly mixed by syringe-to-syringe transfer. The solution was transferred to a glass vial and centrifuged for 5 min at 4000 RPM to remove air bubbles. The resulting gel was allowed to react for 16 hrs at 4 °C. The gel was then particulated through a 100 micron pore-sized mesh. Following sizing, the gel was sterilized by dialysis through a 20 kDa molecular-weight cut-off cellulose ester membrane against 70 % isopropanol / 30 % water for 3 hrs at 4°C. Dialysis was then continued against sterile phosphate buffer, pH 7.4, for 72 hrs at 4°C with four changes of buffer. The gel was then dispensed into syringes under aseptic conditions.

This procedure was used to produce hydrogels with varying concentrations of hyaluronic acid and collagen. When required, human collagen(I) in 0.01 N hydrochloric acid was concentrated from 3 mg/mL to the desired reaction concentration in 20 kDa molecular-weight cut-off centrifugal filtration devices. A 50 mL sample of each gel was synthesized, sterilized by exposure to 70 % isopropanol, and purified by dialysis against phosphate buffer, pH 7.4. The gels synthesized are described in Table 2 along with their rheological properties. Oscillatory parallel plate rheology was used to characterize the rheological properties of gels using an Anton Paar MCR 301. A plate diameter of 25 mm was used at a gap height of 1 mm. The storage modulus (G') and loss modulus (G") were determined at 2% strain and 5 Hz.

**Table 2: Hyaluronic acid-human collagen(I) hydrogel synthesis concentrations and rheological properties**

| **Sample ID** | **[HA] (mg/mL)** | **[Col(I)] (mg/mL)** | **G' (Pa)** | **G" (Pa)** |
|---|---|---|---|---|
| **A** | 3 | 3 | 199 | 24.6 |
| **B** | 12 | 6 | 1260 | 154 |
| **C** | 16 | 8 | 2450 | 288 |
| **D** | 12 | 12 | 3160 | 420 |
| **E** | 24 | 12 | 5440 | 433 |
| **F** | 12 | 3 | 1110 | 52.2 |
| **G** | 16 | 3 | 1490 | 60.6 |
| **H** | 20 | 3 | 1770 | 49.5 |

### Example 13

In order to determine the biopolymer concentration in gels, the weight of the hydrated gel was compared to that of dried gel. A 2 mL sample of gel was weighed and dried by flash-freezing in liquid nitrogen followed by lyophilization at -50 °C and 0.02 Torr. A solution of the appropriate buffer was also weighed and dried in the same fashion to account for salt content of the gel. The total solids content of the gel was calculated by dividing the dry weight by the wet volume, assuming 1 g/mL density for the wet gel, to give a value in mg/mL. The salt solids content was then subtracted from this value to determine the biopolymer concentration in the gel.

**Table 3: Final concentrations of hyaluronic acid-human collagen(I) hydrogels**

| **Sample ID** | **[HA] (mg/mL)** | **[Col(I)] (mg/mL)** | **Final concentration (mg/mL)** |
|---|---|---|---|
| **A** | 3 | 3 | 5.3 |
| **B** | 12 | 6 | 16.3 |
| **C** | 16 | 8 | 19.4 |
| **D** | 12 | 12 | 22.6 |
| **E** | 24 | 12 | 31.6 |

### Example 14

Swelling ratios for gels were determined relative to initial water content and were measured by monitoring the increase in gel mass after equilibration with phosphate buffer. For each gel, approximately 1 mL was injected into a 15 mL Falcon tube and weighed followed by addition of 10 mL of phosphate buffered saline, pH 7.4. The gels were thoroughly mixed with the buffer and vortexed for 30 seconds. The gels were then allowed to equilibrate in the buffer for 48 hrs at 4 °C. After this time, the suspensions were centrifuged at 4000 RPM in a swinging bucket rotor for 5 minutes. The supernatant buffer was then decanted and the weight of the swollen gel was measured. The swelling ratio was determined by dividing the final weight of the swollen gel by the weight of the initial gel.

**Table 4: Swelling ratios of hyaluronic acid-human collagen(I) hydrogels**

| **Sample ID** | **[HA] (mg/mL)** | **[Col(I)] (mg/mL)** | **Swelling ratio** |
|---|---|---|---|
| **A** | 3 | 3 | 1.0 |
| **B** | 12 | 6 | 1.7 |
| **C** | 16 | 8 | 1.7 |
| **D** | 12 | 12 | 1.5 |
| **E** | 24 | 12 | 1.7 |

### Example 15

Hyaluronic acid (800 mg, 2 MDa molecular weight) was dissolved in 50 mL of 8 mg/mL porcine collagen(I) solution in 0.01 N hydrochloric acid. Sodium chloride was added at 0.9 wt% and 2-[morpholino] ethanesulfonic acid was added at 100 mM to the solution and mixed. The hyaluronic acid was allowed to hydrate for 1 hr and the solution was homogenized by syringe-to-syringe mixing. The pH of the solution was adjusted to 5.4 by addition of 1 N sodium hydroxide. 1-Ethyl-3-(3-dimethylaminopropyl) carbodiimide HCI (50 Mm) and N-hydroxysulfosuccinimide sodium salt (50 mM) were added to the hyaluronic acid/collagen solution and quickly mixed by syringe-to-syringe transfer. The solution was transferred to a glass vial and centrifuged for 5 min at 4000 RPM to remove air bubbles. The resulting gel was allowed to react for 16 hrs at 4 °C. The gel was then particulated through a 100 micron pore-sized mesh. Following sizing, the gel was sterilized by dialysis through a 20 kDa molecular-weight cut-off cellulose ester membrane against 70 % isopropanol / 30 % water for 3 hrs at 4 °C. Dialysis was then continued against sterile phosphate buffer, pH 7.4, for 72 hrs at 4 °C with four changes of buffer. The gel was then dispensed into syringes under aseptic conditions.

### Example 16

Subcutaneous bolus injections (1 mL) of sample hydrogels are performed via cannulae through a small incision on the dorsum of nude mice. Samples injected consist of crosslinked hyaluronic acid at 16 mg/mL, crosslinked human collagen(I) at 16 mg/mL, and sample B crosslinked hyaluronic acid-human collagen(I) hydrogel from Example 12. At six weeks, the volumetric duration of the samples is determined along with histological evaluation of cellular in-growth and tissue infiltration. It is found that crosslinked hyaluronic acid has 90 % volume duration, crosslinked human collagen(I) has 30 % volume duration, and crosslinked hyaluronic acid-human collagen (I) has 85 % volume duration. Histological evaluation indicated that crosslinked hyaluronic acid and crosslinked collagen has little to no tissue in-growth, whereas cells and newly deposited extracellular matrix are found throughout the hyaluronic acid-human collagen(I) sample.

Unless otherwise indicated, all numbers expressing quantities of ingredients, properties such as molecular weight, reaction conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained. At the very least, and not as an attempt to limit the application of the doctrine of equivalents to the scope of the claims, each numerical parameter should at least be construed in light of the number of reported significant digits and by applying ordinary rounding techniques.

The terms "a," "an," "the" and similar referents used in the context of describing the invention (especially in the context of the following claims) are to be construed to cover both the singular and the plural, unless otherwise indicated herein or clearly contradicted by context. All methods described herein can be performed in any suitable order unless otherwise indicated herein or otherwise clearly contradicted by context. The use of any and all examples, or exemplary language (e.g., "such as") provided herein is intended merely to better illuminate the invention and does not pose a limitation on the scope of any claim. No language in the specification should be construed as indicating any non-claimed element essential to the practice of the invention.

Groupings of alternative elements or embodiments disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Certain embodiments are described herein, including the best mode known to the inventors for carrying out the invention. Of course, variations on these described embodiments will become apparent to those of ordinary skill in the art upon reading the foregoing description. The inventor expects skilled artisans to employ such variations as appropriate, and the inventors intend for the invention to be practiced otherwise than specifically described herein. Accordingly, the claims include all modifications and equivalents of the subject matter recited in the claims as permitted by applicable law. Moreover, any combination of the above-described elements in all possible variations thereof is contemplated unless otherwise indicated herein or otherwise clearly contradicted by context.

In closing, it is to be understood that the embodiments disclosed herein are illustrative of the principles of the claims. Other modifications that may be employed are within the scope of the claims. Thus, by way of example, but not of limitation, alternative embodiments may be utilized in accordance with the teachings herein. Accordingly, the claims are not limited to embodiments precisely as shown and described.

### Embodiments:

1. A crosslinked macromolecular matrix comprising:
   a hyaluronic acid component;
   a collagen component derived from collagen type I or collagen type III;
   wherein the hyaluronic acid component is crosslinked to the collagen component by a crosslinking component; and
   wherein the crosslinking component comprises a plurality of crosslink units, wherein at least a portion of the crosslink units comprise an ester bond or an amide bond.
2. The crosslinked macromolecular matrix of [1], having a weight ratio of the hyaluronic acid component to the collagen component of about 0.5 to about 7.
3. The crosslinked macromolecular matrix of [1], wherein the hyaluronic acid component has an average molecular weight of about 500,000 daltons to about 10,000,000 daltons.
4. The crosslinked macromolecular matrix of [1], further comprising an aqueous liquid comprising water, sodium chloride at a concentration of about 100 mM to about 200 mM, potassium chloride at a concentration of about 2 mM to about 3 mM, and phosphate buffer at a concentration of about 5 mM to about 15 mM, wherein the pH of the liquid is about 7 to about 8.
5. A composition comprising:
   a hyaluronic acid;
   a collagen;
   a water-soluble coupling agent; and
   a buffer;
      wherein the composition is an aqueous solution.
6. The composition of [5], further comprising an activating agent comprising a triazole, a fluorinated phenol, a succinimide, or a sulfosuccinimide.
7. The crosslinked macromolecular matrix of [5], wherein the hyaluronic acid component has an average molecular weight of about 1,000,000 daltons to about 5,000,000 daltons.
8. The crosslinked macromolecular matrix of [5], wherein the collagen is collagen type I.
9. The crosslinked macromolecular matrix of [5], wherein the collagen is collagen type III.
10. The crosslinked macromolecular matrix of [5], wherein the buffer comprises 2-(N-morpholino)ethanesulfonic acid.
11. A method of crosslinking hyaluronic acid and collagen comprising:
   dissolving a hyaluronic acid and a collagen in an aqueous solution to form an aqueous pre-reaction solution, wherein the aqueous pre-reaction solution further comprises a salt or has a pH less than about 4; and
   modifying the aqueous pre-reaction solution to form a crosslinking reaction mixture comprising:
      the hyaluronic acid;
      the collagen;
      a water soluble carbodiimide;
      an N-hydroxysuccinimide or an N-hydroxysulfosuccinimide; and
      the salt; and
      wherein the crosslinking reaction has a higher pH than the aqueous pre-reaction solution; and
      allowing the crosslinking reaction mixture to react to thereby crosslink the hyaluronic acid and the collagen.
12. The method of [11], wherein modifying the aqueous pre-reaction solution to form a crosslinking reaction mixture comprises increasing the pH of the aqueous pre-reaction solution and allowing a substantial amount of fiber formation to occur before adding the water soluble carbodiimide.
13. The method of [11], wherein modifying the aqueous pre-reaction solution to form a crosslinking reaction mixture comprises adding the water soluble carbodiimide to the aqueous pre-reaction solution before substantially any fiber formation occurs.
14. The method of [11], wherein the salt comprises sodium chloride at a concentration of about 80 mM to about 330 mM in the crosslinking reaction mixture.
15. The method of [11], wherein the water soluble carbodiimide is 1-ethyl-3-(3-dimethylaminopropyl)carbodiimide at a concentration of about 20 mM to about 100 mM in the crosslinking reaction mixture.
16. The method of [11], wherein the crosslinking reaction mixture further comprises a non-coordinating buffer having a concentration of about 10 mM to about 1 M.
17. The method of [11], further comprising particulating the crosslinked macromolecular matrix through a mesh having a pore size of about 10 microns to 100 microns.
18. The method of [11], further comprising sterilizing the crosslinked molecular matrix by dialysis;
   wherein the dialysis is against a sterile phosphate buffer solution comprising phosphate buffer, potassium chloride, and sodium chloride, wherein the sterile phosphate buffer solution is substantially isosmotic with respect to human physiological fluid; and
   wherein the dialysis is through a membrane having a molecular weight cutoff of about 5,000 daltons to about 100,0000 daltons.
19. A crosslinked macromolecular matrix prepared by a process of [11].
20. A soft tissue aesthetic product comprising:
   an aesthetic device having a form suitable for injecting or implanting into human tissue; and
   a label comprising instructions to inject or implant the aesthetic device into human tissue;
      wherein the aesthetic device comprises a crosslinked macromolecular matrix comprising:
      a hyaluronic acid component; and
      a collagen component;
         wherein the hyaluronic acid component is crosslinked to the collagen component by a crosslinking component; and
         wherein the crosslinking component comprises a plurality of crosslink units, wherein at least a portion of the crosslink units comprise an ester bond or an amide bond.
21. The product of [20], wherein the collagen component comprises collagen type I or collagen type III.
22. The product of [20], wherein crosslinked macromolecular matrix has a weight ratio of the hyaluronic acid component to the collagen component of about 1 to about 3.
23. A method of improving an aesthetic quality of an anatomic feature of a human being comprising:
   injecting or implanting an aesthetic device into a tissue of the human being to thereby improve the aesthetic quality of the anatomic feature;
   wherein the aesthetic device comprises a crosslinked macromolecular matrix comprising:
      a hyaluronic acid component; and
      a collagen component;
         wherein the hyaluronic acid component is crosslinked to the collagen component by a crosslinking component; and
         wherein the crosslinking component comprises a plurality of crosslink units, wherein at least a portion of the crosslink units comprise an ester bond or an amide bond.
24. The method of [23], wherein the hyaluronic acid component has an average molecular weight of about 1,000,000 daltons to about 3,000,000 daltons.

## Claims

1. A non-therapeutic method of enhancing or regenerating an anatomic feature of a human being, the method comprising: injecting or implanting an enhancement or regeneration device into a tissue of the human being to thereby enhance or regenerate at least a portion of the anatomic feature, wherein the enhancing or regenerating device comprises a crosslinked macromolecular matrix having a hyaluronic acid component and a collagen component.

2. The method of claim 1, wherein the hyaluronic acid component is crosslinked to the collagen component by using a carbodiimide-based coupling agent, thereby forming a crosslinking component.

3. The method of claim 2, wherein the crosslinking component comprises a plurality of crosslink units, and wherein at least a portion of the crosslink units comprise an ester bond or an amide bond.

4. The method of any preceding claim, wherein the crosslinked macromolecular matrix is dispersed in an aqueous liquid in hydrogel form.

5. The method of any preceding claim, wherein the collagen component is derived from collagen type I or collagen type III.

6. The method of any preceding claim, wherein the crosslinked macromolecular matrix has a weight ratio of the hyaluronic acid component to the collagen component of about 0.5 to about 7, about 1 to about 3, about 1 to about 2, about 1 to about 1, or about 2 to about 1.

7. The method of claim 4, wherein the aqueous liquid comprises water, sodium chloride at a concentration of about 100 mM to about 200 mM, potassium chloride at a concentration of about 2 mM to about 3 mM, and phosphate buffer at a concentration of about 5 mM to about 15 mM, wherein the pH of the liquid is about 7 to about 8.

8. The method of any preceding claim, wherein the crosslinked macromolecular matrix is prepared by the following steps:
dissolving a hyaluronic acid and a collagen in an aqueous solution to form an aqueous pre-reaction solution, wherein the aqueous pre-reaction solution further comprises a salt or has a pH less than about 4;
modifying the aqueous pre-reaction solution to form a crosslinking reaction mixture comprising:
the hyaluronic acid,
the collagen,
a water soluble carbodiimide,
an N-hydroxysuccinimide or an N-hydroxysulfosuccinimide, and optionally the salt, and
wherein the crosslinking reaction has a higher pH than the aqueous pre-reaction solution; and
allowing the crosslinking reaction mixture to react to thereby crosslink the hyaluronic acid and the collagen.

9. The method of claim 8, wherein the hyaluronic acid is present in an amount of about 3 mg/mL to about 100 mg/mL.

10. The method of claim 8 or claim 9, wherein the step of modifying the aqueous pre-reaction solution to form a crosslinking reaction mixture comprises (i) increasing the pH of the aqueous pre-reaction solution and allowing fiber formation to occur before adding the water soluble carbodiimide; or (ii) adding the water soluble carbodiimide to the aqueous pre-reaction solution before any fiber formation occurs.

11. The method of claims 8 to 10, wherein the salt comprises sodium chloride at a concentration of about 80 mM to about 330 mM in the crosslinking reaction mixture.

12. The method of claims 8 to 11, wherein the water soluble carbodiimide is 1-ethyl-3-(3-dimethylaminopropyl)carbodiimide at a concentration of about 20 mM to about 100 mM in the crosslinking reaction mixture.

13. The method of claims 8 to 12, wherein the crosslinking reaction mixture further comprises a non-coordinating buffer having a concentration of about 10 mM to about 1 M.

14. The method of claims 8 to 13, further comprising particulating the crosslinked macromolecular matrix through a mesh having a pore size of about 10 microns to 100 microns.

15. The method of claims 8 to 14, further comprising sterilizing the crosslinked molecular matrix by dialysis;
wherein the dialysis is against a sterile phosphate buffer solution comprising phosphate buffer, potassium chloride, and sodium chloride, wherein the sterile phosphate buffer solution is substantially isosmotic with respect to human physiological fluid; and
wherein the dialysis is through a membrane having a molecular weight cutoff of about 5,000 daltons to about 100,0000 daltons.
